# EUROPEAN PATENT APPLICATION

(11) **EP 3 231 369 A1**
(43) Date of publication of application: **18.10.2017**
(21) Application number: 15868469.6
(22) Date of filing: 17.11.2015
(51) Int. Cl.: A61B 8/08

(54) **ULTRASOUND DIAGNOSTIC DEVICE AND ELASTICITY EVALUATION METHOD**

(30) Priority: 08.12.2014 JP 2014248132
(71) Applicant: Hitachi, Ltd., Chiyoda-ku, Tokyo 100-8280 (JP)
(72) Inventor: YOSHIKAWA Hideki, Tokyo 100-8280 (JP)
(74) Representative: MERH-IP Matias Erny Reichl Hoffmann Patentanwälte PartG mbB
(86) International application number: PCT/JP2015/082206
(87) International publication number: WO 2016/093024

(57) **Abstract**

Provided is an ultrasound diagnostic device having an elasticity evaluation function with which high accuracy and high reproducibility can be achieved while avoiding the influence of internal stresses. The ultrasound diagnostic device comprises: a transceiver 13 which causes a probe 12 for transmitting and receiving ultrasonic waves to transmit and receive first, second, third ultrasonic waves to and from a subject; an image production unit 16 for producing an image on the basis of a received signal related to the first ultrasonic wave; a velocity measurement unit 17 which sets a measurement region on the basis of the produced image, transmits the second ultrasonic wave to the measurement region to create shear waves, and calculates the propagation velocity of the shear waves from a received signal obtained by transmitting and receiving the third ultrasonic wave to and from the measurement region; and an elasticity evaluation unit 18 for evaluating the elasticity of the subject on the basis of the calculated propagation velocity. The velocity measurement unit measures the propagation velocity at the same position a plurality of times. The elasticity evaluation unit 18 derives an elasticity evaluation index using variations in the results obtained by measuring the propagation velocity a plurality of times.

## Description

### Technical Field

The present invention relates to an ultrasound diagnostic device, and a technology of generating a shear wave in an examined body and evaluating elasticity from the propagation velocity of the wave.

### Background Art

An image display device for medical use represented by ultrasonic imaging, MRI (Magnetic Resonance Imaging), or X-ray CT (Computed Tomography) is used widely as a device of exhibiting information in an invisible living body in the form of a numerical value or an image. Among those devices, an image display device using an ultrasonic wave has a temporal resolution higher than other devices, and a function of being able to image a heart during pulsation without blurring.

An ultrasonic wave propagating in a living body as an examined body is mostly classified into a longitudinal wave and a transverse wave and, in many technologies mounted on a product, namely in a technology of imaging a tissue morphology and a technology of measuring a blood velocity, information of a longitudinal wave (sound velocity about 1,540 m/s) is used mainly.

In recent years, a technology such as elastography of evaluating the elastic modulus of a tissue by using a transverse wave (hereunder referred to as a shear wave) draws attention and the clinical application of the technology to a mammary tumor and a chronic liver disease advances.

In the technology, a shear wave is generated in a tissue as a measurement object and elasticity is evaluated from the propagation velocity. Methods for generating a shear wave are roughly classified into a mechanical method and a radiation pressure method. The mechanical method is a method of using a vibrator or the like, giving vibration of about 1 kHz to a body surface, and generating a shear wave, and requires a drive unit acting as a vibration source. In the radiation pressure method in contrast, an acoustic radiation pressure is given into a living body by using a focused ultrasonic wave formed by focusing an ultrasonic wave locally in the interior of a tissue and a shear wave is generated by accompanying tissue displacement. Either of the methods is a technology of measuring a tissue displacement in a living body accompanying the propagation of a generated shear wave by using an ultrasonic wave and evaluating information on the hardness of the tissue.

As citations related to the technologies, there are Patent Literature 1 and Patent Literature 2 related to a method of evaluating elasticity by using acoustic radiation pressure for example.

### Citation List

### Patent Literature

PTL 1: Japanese Patent Application Laid-Open No. 2010-526626
PTL 2: Japanese Patent Application Laid-Open No. 2014-064912

### Non-patent Literature

NPTL 1: "Acoustoelasticity", Incorporated Association: Japanese Society for Non-destructive Inspection, P2 - P15 (published in November 20, 1994)

### Summary of Invention

### Technical Problem

In the method described in Patent Literature 1, radiating power is generated in a tissue by using a focused ultrasonic wave and a shear wave is propagated in the tissue. A plurality of measurement points for transmitting and receiving an ultrasonic wave are prepared in a propagation direction and the temporal variation of tissue displacement is measured. By using the measurement result of the displacement, an arrival time of the shear wave is measured at each of the measurement points. By using the arrival time at each of the measurement points, a propagation time of the shear wave between the measurement points is calculated and a velocity is measured.

The method described in Patent Literature 2 relates to strain imaging using static elastography of oppressing a test object from an exterior and displaying displacement generated in the test object as a two-dimensional image, and relates to a method of detecting an optimum compression state. On this occasion, the strength of the compression from the exterior is monitored continuously by using the displacement, for example a time phase allowing a maximum displacement is selected from the result, and the time phase is judged as a time phase optimum to the strain imaging.

It is known in the field of non-destructive inspection of concrete or the like that, when force is applied to a medium in which a shear wave propagates from an exterior, elastic deformation, namely internal stress, is generated in the interior of the medium and influences an acoustic characteristic (a propagation velocity for example) of the shear wave (refer to Non-patent Literature 1). Specifically, the document says that there is a linear relationship between a propagation velocity and a stress (or a strain approximately) and the proportionality coefficient is described by a high-order elastic constant including a three-dimensional elastic constant. Moreover, it is known that the proportionality coefficient has a direction dependence on the direction of a force (stress axis) and varies in response to the propagation direction of a shear wave and the direction of medium vibration.

A similar phenomenon is surmised also in a soft tissue of a living body and the possibility of including an internal stress caused by an external force from a surrounding tissue is conceivable. When a blood vessel or a tubercle exists on a propagation route of a shear wave, the shear wave is influenced by diffraction and refraction. The existence of such a tissue structure is a "visible" error factor that can be confirmed by image information or the like. In contrast, the variation of an acoustic characteristic caused by an internal stress is an "invisible" error factor, does not have an avoidance means based on information such as an image or the like, and hence is an important problem relating not only to the deterioration of accuracy in elasticity evaluation function but also to the deterioration of reproducibility. In the case of a test object having a homogeneous elastic modulus for example, the velocity distribution of a shear wave is constant regardless of a region of the test object. When an internal stress differs in accordance with a region however, the velocity distribution of a shear wave also differs, a result different from an actual situation is derived, and diagnostic accuracy is influenced.

An object of the present invention is to provide an ultrasound diagnostic device and an elasticity evaluation method, which allow elasticity evaluation with high degrees of accuracy and reproducibility by using the variation of an acoustic characteristic caused by an internal stress.

### Solution to Problem

The present invention, in order to attain the above object, provides an ultrasound diagnostic device comprising a transceiver to transmit and receive first, second, and third ultrasonic waves to and from a test object through a probe to transmit and receive an ultrasonic wave, an image generation unit to generate an image of the test object by a received signal based on the first ultrasonic wave, a velocity measurement unit to transmit the second ultrasonic wave to a measurement region set on the basis of the image, generate a shear wave, and measure the propagation velocity of the shear wave by the third ultrasonic wave, and an elasticity evaluation unit to evaluate the elasticity of the test object on the basis of the propagation velocity, wherein the velocity measurement unit measures the velocity more than once at an identical site, and the elasticity evaluation unit derives an elasticity evaluation index of the test object by using the variation of the velocity measurement results.

Further, the present invention, in order to attain the above object, provides an elasticity evaluation method using a probe, comprising the processes of generating a shear wave in a measurement region of a test object, measuring the propagation velocity of the shear wave in the measurement region more than once at an identical site by transmitting and receiving an ultrasonic wave to and from the probe, and deriving an elasticity evaluation index of the test object by using the variation of a plurality of obtained velocity measurement results.

### Advantageous Effects of Invention

The present invention makes it possible to derive an elasticity evaluation index with a high degree of accuracy and a good reproducibility.

### Brief Description of Drawings

Figure 1 is a block diagram showing a configuration example of an ultrasound diagnostic device according to Example 1.
Figure 2 is a view showing a flowchart of processing at an elasticity evaluation unit according to Example 1.
Figure 3A is a view showing an example of a method of setting a measurement region according to Example 1.
Figure 3B is a view showing an example of a test object and an external force source according to Example 1.
Figure 4A is a view showing an electrocardiogram and time points of velocity measurement according to Example 1.
Figure 4B is a view showing a relationship between a stress index and a shear wave velocity according to Example 1.
Figure 5 is a view showing a second means on the conversion of a stress index according to Example 1.
Figure 6A is a view sowing compatibility of wave-front measurement and displacement measurement according to Example 1.
Figure 6B is a view showing a transmission sequence and a utilization method of ultrasonic waves according to Example 1.
Figure 7 is a view showing an elasticity evaluation index according to Example 1.
Figure 8A is a view showing an example of an image of a conventional velocity distribution.
Figure 8B is a view showing an example of an image of an elasticity evaluation index distribution according to Example 1.
Figure 8C is a view showing an example of an image of a variation of an elasticity evaluation index according to Example 1.
Figure 9 is a block diagram showing a configuration of an ultrasound diagnostic device according to Example 2.
Figure 10 is a view showing a flowchart leading to elasticity evaluation according to Example 2.
Figure 11 is a view showing symmetry of velocity vectors according to Example 2.
Figure 12 is a view showing First Example of two-direction transmission according to Example 2.
Figure 13 is a view showing Second Example of two-direction transmission according to Example 2.
Figure 14 is a view showing an example of an elasticity evaluation result according to Example 2.
Figure 15 is a view showing an example of a display form according to Example 2.
Figure 16 is a view showing a method of calculating a stress index according to Example 2.
Figure 17 is a view showing a configuration of an ultrasound diagnostic device according to Example 3.
Figure 18 is a view showing First Example of a method of determining transmission directions according to Example 3.
Figure 19 is view showing Second Example of a method of determining transmission directions according to Example 3.
Figure 20 is view showing Third Example of a method of determining transmission directions according to Example 3.
Figure 21 is a view showing a transmission and reception sequence and a utilization method of ultrasonic waves according to Example 3.
Figure 22 is a view showing a configuration of an ultrasound diagnostic device according to Example 4.
Figure 23 is a view showing a flowchart leading to elasticity evaluation according to Example 4.
Figure 24 is a view showing an example of an elasticity evaluation result according to Example 4.
Figure 25 is a view showing an example of the display of elasticity evaluation results according to the examples.

### Description of Embodiments

Embodiments according to the present invention are explained hereunder in accordance with drawings. Here, in the present description, an elasticity evaluation index that is numerical information involved in the elasticity of a test object indicates a general physical property, such as a displacement, a strain, a particle velocity, a group velocity, a phase velocity, an attenuation rate, a Young's modulus, a rigidity modulus, a complex modulus (storage elastic modulus, loss elastic modulus), a volume elasticity modulus, a shear wave velocity, a longitudinal wave velocity, a viscosity rate, or a Poisson's ratio, of a tissue but the following explanations are based on a shear wave velocity.

Further, in the present description, a method of exciting a shear wave indicates a general vibration source, such as an acoustic radiation pressure, a mechanical vibration of a vibrator or the like, or a cardiac pulsation, that gives vibration to a test object but the following explanations are bases on a method of using an acoustic radiation pressure. The present invention however is not limited to the method. Furthermore, in the present description, an external force that gives an internal stress to a test object indicates a general external force source, such as mechanical vibration using a vibrator, oppression using a probe, or a pressure accompanying organ motion such as cardiac pulsation, applied to the test object but the following explanations are based on an external force caused by cardiac pulsation. Moreover, in the present description, a test object indicates a general medium in which a shear wave propagates, and is basically a living tissue, but is not limited in particular. The following explanations are based on a liver texture. In addition, in the present description, an index related to an internal stress of a test object, namely a stress, a displacement, a strain, a particle velocity, a time corresponding to the magnitude of a stress, or the like, is generically called a stress index.

### <Example 1 >

Example 1 is an example of an ultrasound diagnostic device configured so as to comprise a transceiver 13 to transmit and receive first, second, and third ultrasonic waves to and from a test object through a probe to transmit and receive an ultrasonic wave, an image generation unit 16 to generate an image of the test object by a received signal based on the first ultrasonic wave, a velocity measurement unit 17 to transmit the second ultrasonic wave to a measurement region set on the basis of the image, generate a shear wave, and measure the propagation velocity of the shear wave by the third ultrasonic wave, and an elasticity evaluation unit 18 to evaluate the elasticity of the test object on the basis of the propagation velocity, wherein the velocity measurement unit 17 measures the velocity more than once at an identical site, and the elasticity evaluation unit 18 derives an elasticity evaluation index of the test object by using the variation of the velocity measurement results. Further, Embodiment 1 is an example of an elasticity evaluation method using a probe, comprising the processes of generating a shear wave in a measurement region of a test object, measuring the propagation velocity of the shear wave in the measurement region more than once at an identical site by transmitting and receiving an ultrasonic wave to and from the probe, and deriving an elasticity evaluation index of the test object by using the variation of a plurality of obtained velocity measurement results.

That is, the present example is an example of outputting an elasticity evaluation index of a test object on the basis of a plurality of shear wave propagation velocities measured in the states of different time phases and different internal stresses. Further, the present example is an example of an ultrasound diagnostic device configured so that the ultrasound diagnostic device further comprises a stress index measurement unit 20 and the stress index measurement unit 20 measures the variation of the test object accompanying an external force as a stress index by using a received signal based on the transmission of the first ultrasonic wave or the third ultrasonic wave, and an elasticity evaluation method of measuring the variation of the test object accompanying an external force as a stress index by using a received signal based on the transmission of the first ultrasonic wave or the third ultrasonic wave.

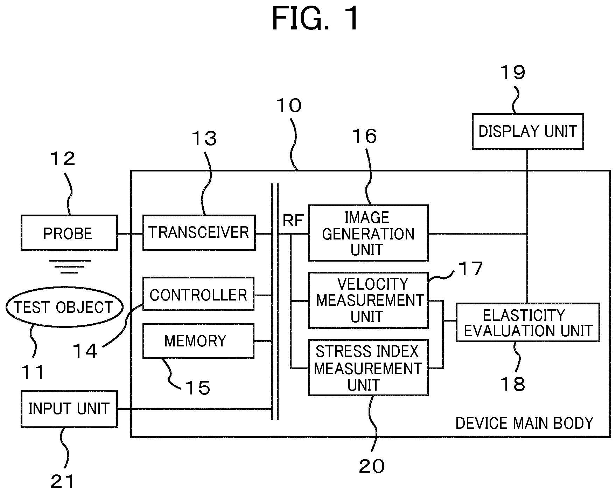

An ultrasound diagnostic device and an elasticity evaluation method according to the present example are explained in reference to a block diagram showing a configuration example of an ultrasound diagnostic device in Figure 1. In the configuration of the ultrasound diagnostic device in Figure 1, a device main body 10 is a collective term of a whole block that includes a velocity measurement unit 17 to transmit and receive second and third ultrasonic waves to and from a measurement region in an examined body and measure a velocity of a shear wave and further an elasticity evaluation unit 18 to measure velocities more than once in different time phases and derive an elasticity evaluation index on the basis of the result, and processes signals on the basis of RF (Radio Frequency) data or video data. As it will be explained later, the device main body 10 can be configured by using a computer such as an ordinary personal computer (PC) except a transceiver 13.

A configuration related to image generation used in the present example is explained firstly. A method of transmitting and receiving an ultrasonic wave related to image generation carried out through a probe 12 is a generally known technological content and hence is explained briefly by omitting the details. As shown in Figure 1, an electric signal for a transmission pulse is transmitted from a transceiver 13 of an ultrasonic wave, namely a transmission beamformer (transmission BF) to generate an ultrasonic signal, to a probe 12 that is installed over the body surface of a test object 11 that is an examined body and transmits and receives an ultrasonic wave through a digital-to-analog (D/A) converter. The electric signal inputted into the probe 12 is converted from the electric signal to an acoustic signal by a ceramic element installed in the interior and the acoustic signal is transmitted to the test object. The transmission is carried out by a plurality of ceramic elements and predetermined time delays are given to the respective elements so that the signals may focus on the test object 11 that is the examined body in a predetermined depth.

The acoustic signal reflected during the course of propagating in the interior of the test object is received again by the probe 12 and sent to the transceiver 13. The acoustic signal is converted into an electric signal in the interior of the transceiver 13 contrary to the time of transmission and the electric signal passes through an analog-to-digital (A/D) converter and is sent to a reception beamformer (reception BF) to generate a complex RF data from the received ultrasonic signal as received data. At the reception BF, phasing addition that is addition processing considering time delay given during transmission is applied to the signals received by the elements, processing such as attenuation correction is applied, and successively complex RF data are generated. The complex RF data are sent to an image generation unit 16 to generate a brightness mode image (B image) showing the morphologic information of a tissue. As explained above, in Figure 1, a series of blocks including the transmission BF, the D/A converter, the A/D converter, and the reception BF and being involved in the transmission and reception of an ultrasonic signal are collectively called a transceiver 13 and the control is carried out by a controller 14. As the controller 14, a central processing unit (CPU) incorporated into a PC or the like can be used. A memory 15 similarly uses a storage unit incorporated into a PC or the like and stores various kinds of programs implemented at the controller 14 and various kinds of obtained data.

The RF data outputted from the transceiver 13 come to be element data of a specific one line along transmitting and receiving directions of an ultrasonic wave in image data finally displayed on a display unit 19. By transmitting and receiving an ultrasonic wave to and from the test object while switching sequentially in the arrangement direction of the ceramic elements constituting the probe 12, RF data are obtained as all the received data coming to be constituent elements of image data and displayed on the display unit 19. The display unit 19 can use a display for exclusive use or a display such as a PC and, as it will be explained later, is also used for displaying a result of elasticity evaluation derived by the elasticity evaluation unit 18 in the form of a numerical value, a graph, or an image.

At the image generation unit 16, the RF data that are the received data obtained from the transceiver 13 is subjected to image generation processing, such as gain control, logarithmic compression, envelope demodulation, or pixel interpolation or coordinate conversion responding to the type of a probe, generally used in a prevailing ultrasound diagnostic device and a B image showing morphologic information in the interior of the test object is generated. The image generation unit 16 can comprise a CPU in a PC or the like or an image processing integrated circuit (IC) for exclusive use similarly to the controller 14 described earlier. The generated B image is displayed on the display unit 19 to display an acquisition condition of a signal, an image, and a numerical value.

A device main body 10 according to the present example, as shown in Figure 1, comprises a velocity measurement unit 17 to generate a shear wave in a test object 11 and measure a propagation velocity of the shear wave, an elasticity evaluation unit 18 to evaluate an elasticity of the test object 11 on the basis of the measured propagation velocity, and further a stress index measurement unit 20 to measure a stress index such as a stress, a displacement, a strain, a particle velocity, or a time corresponding to the magnitude of a stress, those being indexes related to an internal stress of the test object 11. The velocity measurement unit 17, the elasticity evaluation unit 18, and the stress index measurement unit 20 can be operated by running a program in a CPU in a PC explained earlier.

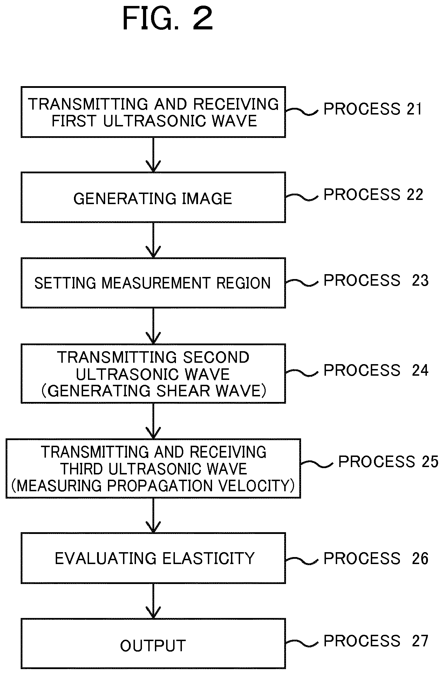

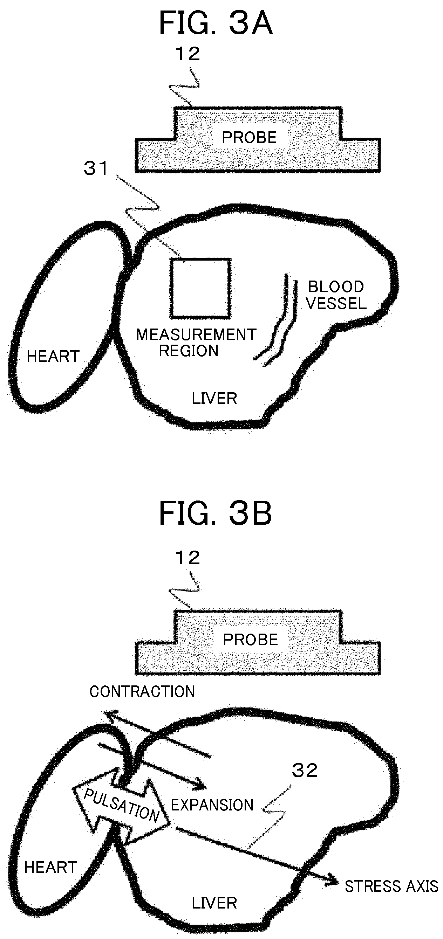

Successively, by using Figures 2 and 3A, a flowchart of operation processing from setting a measurement region to evaluating elasticity in an ultrasound diagnostic device according to the present example is shown. Firstly a first ultrasonic wave is transmitted and received through a transceiver 13 at Process 21 and a B image of a test object is generated at Process 22. Successively, an operator that is a user selects a measurement region 31 where elasticity is evaluated on the basis of the B image displayed on a display unit 19. The position of the measurement region 31 can be selected by using an input unit 21 such as a keyboard or a mouse, those being input means of a PC shown in Figure 1.

Here, the size of the measurement region 31 is decided in response to a propagation range of a shear wave. That is, with regard to the depth direction, a controller 14 sets the range so as to be comparable with the distribution range of an acoustic radiation pressure and determines mostly by an aperture, a frequency, and the like, those being transmission conditions of a second ultrasonic wave. With regard to an azimuth direction, likewise the controller 14 determines the range mostly by the attenuation of a generated shear wave, namely a wave-front amplitude, and the sensitivity of velocity measurement. By increasing the transmission acoustic pressure of the second ultrasonic wave, a shear wave of a large amplitude is generated and a propagation distance increases.

Because temperature rise is induced in a living body and safety is not secured however, the transmission acoustic pressure and wave train length (wave number) of a second ultrasonic wave are limited from the viewpoint of safety. In general, safety and measurement sensitivity is compatible with each other when a propagation distance is about 10 mm. Further, with regard to a site where a measurement region is set, because a construction such as a blood vessel or a tubercle disturbs the wave front of a shear wave by the effects of diffraction, refraction, and reflection and causes velocity measurement, a measurement region 31 is set at a site where such a construction is avoided as shown in Figure 3A.

Subsequently, the generation of a shear wave and velocity measurement are carried out by a velocity measurement unit 17 at Process 24 and Process 25 in Figure 2. The velocity measurement unit 17 generates a shear wave and measures the propagation velocity by using second and third ultrasonic waves. Firstly an acoustic radiation pressure is excited in the interior of a test object by the transmission of the second ultrasonic wave and a local displacement is generated. As the second ultrasonic wave, a burst wave having a wave train length (wave number) larger than an ultrasonic signal used for generating a B image is generally used. At the same time as the transmission of the second ultrasonic wave stops, a restorative force to try to restore the local displacement acts in a medium. With a shear stress accompanying the restorative force used as a starting point, a shear wave propagates in a direction (radial direction) perpendicular to the transmission direction of the second ultrasonic wave.

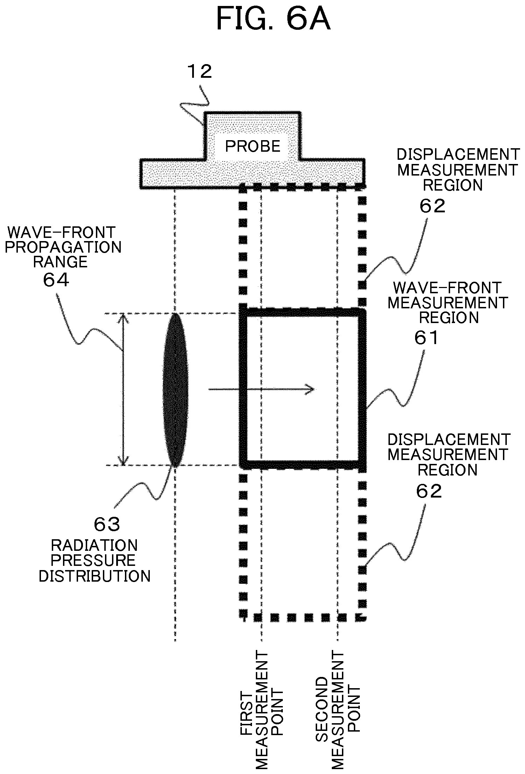

The shear wave advances while vibrating the medium in the direction perpendicular to the propagation direction, namely in the direction parallel with the transmission direction of the second ultrasonic wave. By setting a plurality of measurement points in the propagation direction and measuring the arrival times of the shear wave, a distance between the respective measurement points and a time difference between the arrival times are obtained and resultantly the propagation velocity of the shear wave is calculated. Here, as seen in Figure 6A, in the present explanations, two measurement points are assumed and are called a first measurement point and a second measurement point. An arrival time is measured by using the transmission of the third ultrasonic wave. The transmission conditions of the third ultrasonic wave including acoustic parameters such as a frequency, a wave number, and an F number are nearly identical to the conditions when image data are generated. When the test object is an abdomen, the conditions of a frequency of 1 to 5 MHz, a wave number of 1 to 3, and an F number of 1 to 2 are used. The displacement of the medium for detecting a shear wave is measured by using a received signal (RF data) to the transmission of the third ultrasonic wave and a complex correlation calculation such as a Doppler method or a speckle tracking method.

Specifically, firstly an ultrasonic wave is continuously transmitted and received and the temporal variation of a medium displacement is observed at the first measurement point. Since the medium displacement varies largely together with the arrival of a wave front to the measurement point in a wave-front measurement region, an arrival time is calculated from an extreme value, a zero-crossing point, or the like of a wave front, those being quantities characteristic to a wave front. At the second measurement point too, processing similar to the first measurement point is carried out and a wave-front arrival time to the second measurement point is calculated. Although the wave-front measurement is assumed to be carried out by the medium displacement in the above explanations, the medium displacement is also thought to be a particle velocity of a medium if it is captured as a medium displacement in a repetition frequency (pulse repetition time: PRF) of pulse transmission that is a time required for transmitting and receiving an ultrasonic wave. In the wave-front measurement besides, there is no specific limitation as long as a medium variation that can detect propagation exists and in any case an arrival time can be calculated without any difficulty.

Elasticity is evaluated at Process 26 on the basis of a measured propagation velocity. As shown in Figure 3B, a liver is close to a heart and receives a stress that is an external force caused by cyclic pulsation movement of contraction and expansion in the direction of a stress axis 32. In the environment, when velocity measurement is carried out more than once, velocities in different pulsation time phases (namely, under different strains) are calculated. In the elasticity evaluation method according to the present example, velocity measurement is carried out more than once and an elasticity evaluation index (namely, a high-order elastic constant showing the nonlinearity of elasticity and an index related to it) is derived in consideration of velocity variation under different stresses.

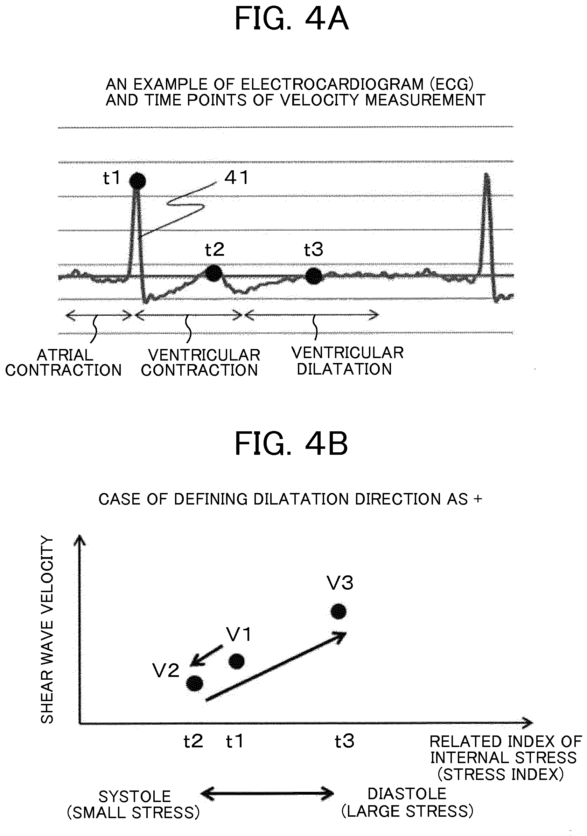

For ease of explanation, explanations are made in reference to the schematic views of Figures 4A and 4B. When pulsation movement is expressed in an electrocardiogram (ECG), like an ECG waveform 41 in Figure 4A, a cyclic movement comprising atrial contraction, ventricular contraction, and then ventricular dilatation is repeated. When a perspective is focused on a liver, at a systole where a heart contracts, an external force reduces and an internal stress lowers. On the other hand, at a diastole where a heart expands, a reverse phenomenon occurs, namely an external force increases and an internal stress also increases. When velocity measurement is carried out at three time points (t1, t2, and t3) on an electrocardiogram for example, the corresponding measured velocities (V1, V2, and V3) are graphed as shown in Figure 4B in the state where the stress is small at the velocity V2 corresponding to the utmost systole and the stress is large at the velocity V3 corresponding to the utmost diastole. The vertical axis in the graph represents a shear wave velocity and the horizontal axis represents a stress index that is an index related to an internal stress. Here, the shear wave velocity is assumed to increase during a diastole. An electrocardiogram is discriminated by waveforms (P, Q, R, S, T, and U) in accordance with the behavior of a ventricle and an atrium and relative magnitude relationship in stress states where the velocity measurements are carried out can be measured by using the waveforms.

On the horizontal axis of the graph in Figure 4B, a stress, a displacement, a strain, a particle velocity, a time corresponding to the magnitude of a stress, or the like, those being stated earlier, is set as a stress index related to an internal stress. The stress index is derived by a stress index measurement unit 20 and a plurality of means can be considered as the calculation method. A first means is a method of judging the relative magnitude of an internal stress by using the three time points (t1, t2, and t3) on an electrocardiographic waveform as stated above. That is, the stress index measurement unit 20 measures the variation of a test object 11 accompanying an external force as a stress index by using a received signal based on the transmission of a first ultrasonic wave or a third ultrasonic wave from a probe 12. Further, the stress index measurement unit 20 derives a stress index on the basis of an electrocardiographic waveform of a test object.

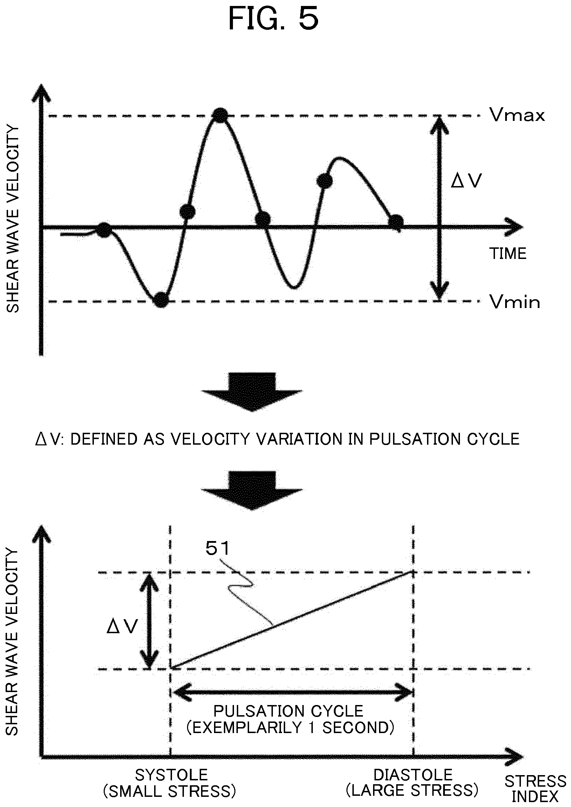

Successively, a method of not using an electrocardiogram is explained as a second means of stress index calculation by the stress index measurement unit 20 according to the present example in reference to Figure 5. In the second means, an elasticity evaluation unit evaluates the relationship between a plurality of propagation velocities measured by a velocity measurement unit 17 and stress indexes corresponding to the respective propagation velocities with an approximate straight line, and defines an inclination angle or a velocity variation of the approximate straight line as an elasticity evaluation index.

When the velocity of a shear wave is measured intermittently more than once in a liver under cyclic pulsation, a measurement result of velocity varying periodically is obtained as shown at the upper stage in Figure 5. The maximum velocity (Vmax) and the minimum velocity (Vmin) can be regarded as velocities corresponding to the maximum and the minimum of an internal stress caused by pulsation. By measuring a pulsation cycle of an examined body beforehand for example therefore, a waveform 51 in the graph of the relationship between a stress index and a velocity variation is configured as shown at the lower stage in Figure 5. The pulsation cycle is supposed to coincide with the cycle of a velocity measurement result and the velocity measurement and the measurement of a pulsation cycle can be carried out simultaneously.

As a third means of calculating a stress index by the stress index measurement unit 20, an example of directly evaluating an internal stress and measuring a displacement of a measurement region is explained. As a method of measuring a displacement, a general method of measuring the travel distance of a medium such as complex correlation calculation or speckle tracking is used. When a single-dimensional signal such as RF data is used, a measured displacement is the component of an actual displacement projected in the transmitting and receiving direction of an ultrasonic wave. When a two-dimensional signal such as a B image is used, a displacement along a stress axis that is a stress direction can be measured and hence displacement measurement of a high sensitivity can be carried out in comparison with the case of using a single-dimensional signal.

The transmission and reception of an ultrasonic wave in an ultrasound diagnostic device according to the present example is roughly categorized into the transmission and reception aimed at B image configuration and the transmission and reception aimed at the velocity measurement of a shear wave. In order to observe a displacement caused by pulsation having periodicity of several seconds, the displacement has to be observed as continuously as possible including the time zone of velocity measurement. At the stage of velocity measurement however, a displacement accompanying the propagation of a shear wave overlaps in addition to a displacement derived from pulsation and hence a certain restriction is added. Firstly, in the irradiation time zone of a second ultrasonic wave for exciting an acoustic radiation pressure, a burst wave is used and hence displacement measurement cannot be carried out and is evaded.

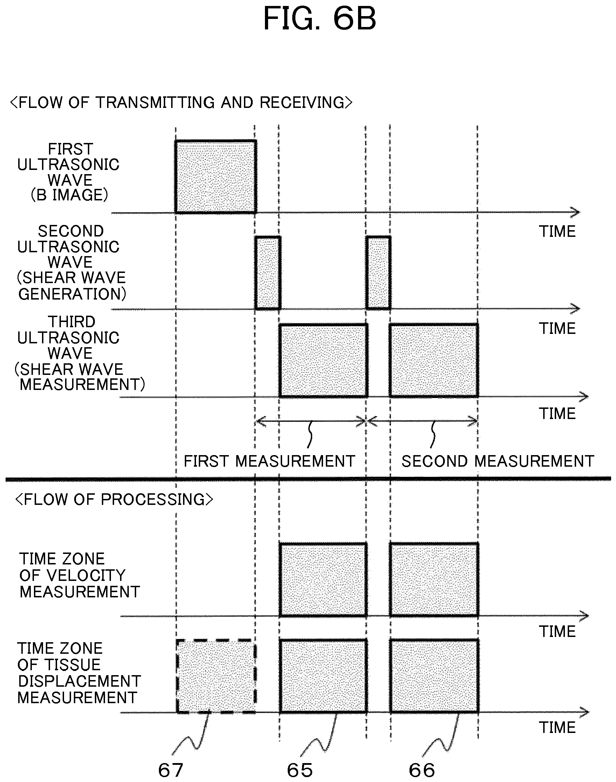

As shown in Figure 6A and Figure 6B, displacement measurement can be carried out in the irradiation time zone of the third ultrasonic wave to measure the propagation of a shear wave. Since an acoustic radiation pressure has a radiation pressure distribution 63 in a finite range of the depth direction, the wave-front propagation range 64 of the shear wave is also limited in the range. The measurement region 31 in Figure 3A set on the basis of a B image is set roughly in the propagation range. Consequently, the regions of a shallow part and a deep part located in front and in the rear of a wave-front measurement region 61 when viewed from a probe 12 are not susceptible to the shear wave and hence can be set as a displacement measurement region 62 to measure a displacement derived from pulsation. A burst wave that is the second ultrasonic wave to generate the shear wave is generally in the range from 0.1 millisecond to 1 millisecond and is a very short time in comparison with a pulsation cycle. By the above method therefore, as continuous displacement measurement as possible can be realized from the stage of the generation of a B image to the stage of velocity measurement. Further, it shows that, when the stress index measurement unit 20 measures the variation of a test object accompanying an external force by using a received signal based on the transmission of the third ultrasonic wave as a stress index, the measurement is preferably carried out on the basis of a received signal from a region obtained by excluding a region where the shear wave propagates from a region where the third ultrasonic wave propagates.

The relationship between the flow of transmitting and receiving ultrasonic waves and the flow of processing of velocity measurement and displacement measurement corresponding to the flow in a device according to the present example is shown in Figure 6B. As shown in the flow of transmitting and receiving at the upper stage in Figure 6B, the transmission or reception of the first, second, and third ultrasonic waves is carried out continuously and the first measurement and the second measurement in the time zone of velocity measurement correspond to the time zone of the reception of the third ultrasonic wave. An important viewpoint is a time zone corresponding to displacement measurement. As shown at the lower stage in the figure, the time zones 65 and 66 of tissue displacement measurement are set in parallel with the time zone of the transmission and reception of the third ultrasonic wave, namely the velocity measurement, and the displacement measurement is carried out.

Further, as shown by the time zone 67 in the broken line frame, the time zone of the transmission and reception of the first ultrasonic wave at the B image generation stage can also be utilized for tissue displacement measurement. An object of tissue displacement measurement is to evaluate the state of a medium at the time point when a shear wave propagates and hence the displacement measurement in this time zone is not an essential process for carrying out the elasticity evaluation method according to the present example. The overall background information of a medium obtained by the displacement measurement in this time zone however is important for correlating a propagation velocity with a tissue displacement with a higher degree of accuracy.

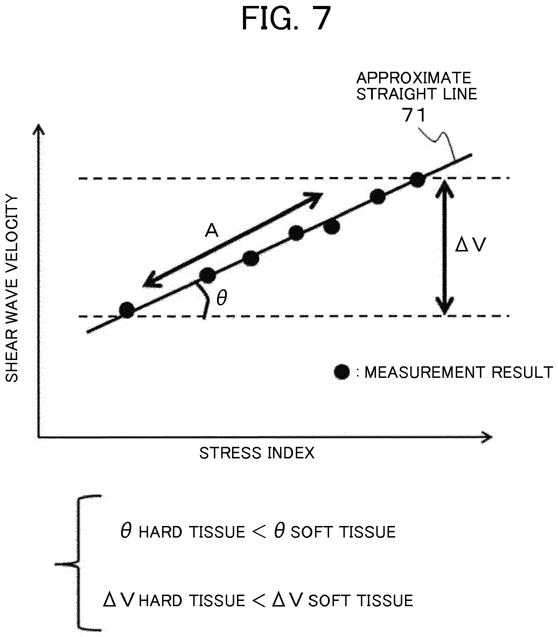

An elasticity evaluation index is derived by an elasticity evaluation unit 18 from a graph of the relationship between a stress index obtained by the configuration explained above and a shear wave velocity. An approximate straight line 71 showing the relationship between a stress index and a shear wave velocity is shown in Figure 7. As shown in the figure, a velocity variation ΔV or an inclination angle θ of the approximate straight line 71 is named as an elasticity evaluation index. When the hardening of a liver tissue advances by the progress of a liver disease such as fibrosis, an internal stress accompanying pulsation lowers or a displacement lowers and hence the velocity variation lowers although the absolute value of the velocity increases. At the same time, the inclination angle θ also becomes acute. By comparing elasticity evaluation indexes between before and after medical treatment therefore for example, information to support the judgment of the effect of the treatment is provided. In other words, it shows that the elasticity evaluation unit 18 appropriately evaluates the relationship between a plurality of propagation velocities measured by the velocity measurement unit 17 and the strain indexes corresponding to the respective propagation velocities through the approximate straight line, and sets the inclination angle or the velocity variation as an elasticity evaluation index.

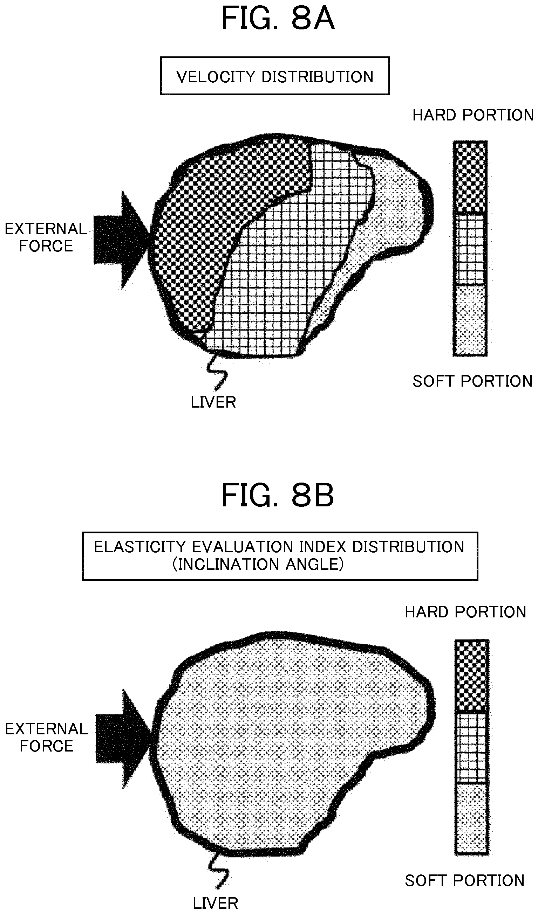

In prior art, the propagation velocity of a shear wave has been used directly as an elasticity evaluation index. On this occasion, a propagation velocity varies in response to the magnitude of an internal stress, namely a propagation velocity varies in the vertical directions in accordance with the variation in the directions of the arrow A in Figure 7 and a difference appears in a velocity distribution even in a test object actually having a homogeneous elastic modulus. Figure 8A is a view showing the influence of an internal stress that is an external force on a liver in prior art. Further, when an internal stress varies periodically or non-periodically, a velocity distribution varies in accordance with a time phase of measurement and both accuracy and reproducibility deteriorate.

By a method according to the present example in contrast, a velocity variation or an inclination angle caused by the magnitude of an internal stress is used as an elasticity evaluation index. By a velocity variation, the influence on a measurement time phase can be mitigated but the influence of difference in internal stress between regions cannot be evaded. When the range for elasticity evaluation of a liver is small, difference in internal stress between regions is small and hence the use of a velocity variation is also effective. On the other hand, with regard to an inclination angle, since the inclination angle is kept constant even when an internal stress varies, namely since an angle θ of the inclination angle does not vary even when an external force varies in the directions of the arrow in Figure 7, when an inclination angle distribution is configured as a two-dimensional image as shown in Figure 8B, a homogeneous distribution image is obtained regardless of an internal stress. That is, by the elasticity evaluation method according to the present example, the provision of diagnostic information of high degrees of accuracy and reproducibility can be realized.

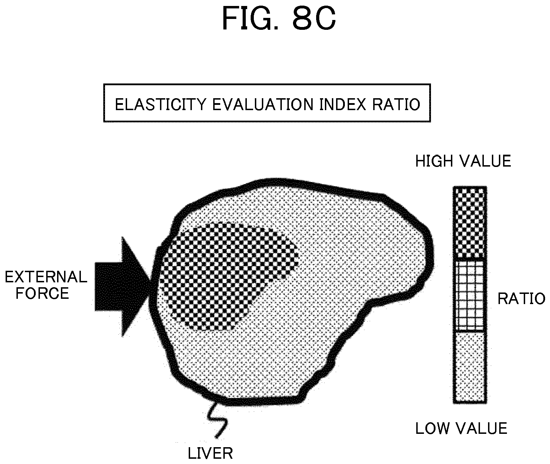

Moreover, the variation of an elasticity evaluation index accompanying therapeutic processes is also important clinically. To this end, a display mode (Figure 8C) obtained by taking the ratio of elasticity evaluation index distributions before and after medical treatment and changing coloration in response to the magnitude of a numerical value is also effective. On this occasion, a similar clinical effect is obtained as long as the ratio (relative ratio) of elasticity evaluation indexes, including inclination angles or velocity variations ΔV in Figure 7, before and after medical treatment is used.

In an ultrasound diagnostic device and an elasticity evaluation method according to the present example explained above, the stress axis 32 in Figure 3B in the direction of an internal stress is not taken into consideration. Actually, with regard to velocity variation accompanying an internal stress, the increase or decrease (acceleration or deceleration) or the quantitative content depends on the direction of wave-front propagation and the direction of wave-front vibration relative to a stress axis. Consequently, the present example is the one related to a method of deriving a simple elasticity evaluation index not requiring the identification of a stress axis. Since this is a technology of using a velocity variation relative to an internal stress as an elasticity evaluation index however, in the case of comparing between before and after medical treatments for example, the influence of a stress axis is preferably reduced by integrating the installation site of a probe and a positional relationship between a measurement region and a heart to the greatest possible extent.

Although the explanations are described on the premise that the test objet is a liver in Example 1 stated above, the test object is not particularly limited as long as it is a tissue in a living body. With regard to an external force source too, the external force is not particularly limited as long as it is a factor acting on a test object as an external force, such as the pulsation of a heart, a mechanical vibration from a body surface, a blood vessel inner pressure, the peristaltic motion of a digestive tube, or the inner pressure of an abdominal cavity.

Further, the present example can be implemented as long as the used probe 12 is a device that can transmit and receive an ultrasonic wave and no limitation is particularly set on the types of a probe including a linear type, a convex type, and a sector type. For example, by using a probe having a two-dimensional array, the velocity of a shear wave can be measured in an arbitrary direction in a space. Furthermore, with regard to a method of generating a shear wave, although a method of using the acoustic radiation pressure of the second ultrasonic wave is shown as an example in the present example, no limitation is particularly set as long as the method is a method that can generated a shear stress in a test object, such as a method of using a device such as a vibrator.

In addition, with regard to a display mode, although the modes of two-dimensional images are exemplified in Figures 8A, 8B, and 8C, a mode of measuring an elasticity evaluation index in a specific region of a test object and displaying it as numerical information is also included in the display mode according to the present example.

In this way, according to Example 1 described in detail, an evaluation index on the elasticity of a test object is derived from the relationship between an internal stress and a velocity in a liver or the like and an ultrasound diagnostic device having an elasticity evaluation function of high degrees of accuracy and reproducibility can be realized.

### <Example 2>

Example 2 is an example of an ultrasound diagnostic device having the function of extending the content of Example 1, propagating shear waves in a plurality of directions which are not parallel with each other, and deriving an elasticity evaluation index from velocity variations in the respective directions. That is, a device according to the present example is an example configured so that the device further comprises a transmission direction control unit 91 to control transmission directions of an ultrasonic wave, the transmission direction control unit 91 determines a plurality of directions not parallel with each other and transmits a second ultrasonic wave to the directions, and a velocity measurement unit 17 measures the propagation velocities of shear waves corresponding to transmission in the directions. Further, the device according to the present example is an example configured so that an elasticity evaluation unit 18 evaluates relationship between a plurality of propagation velocities measured by the velocity measurement unit 17 and stress indexes corresponding to the respective propagation velocities with approximate straight lines, and outputs a velocity numerical value at the intersection of the approximate straight lines involved in the respective propagation directions as an elasticity evaluation index. Although the explanations are made on the basis of the case of two directions here, the number of the directions is not limited to the case.

Here, the purpose of using the velocity variations of shear waves in a plurality of directions in the present example is to derive an elasticity evaluation index from which the influence of an internal stress is excluded. As stated earlier, the acoustic characteristic of a shear wave varies in response to a propagation direction and a vibration direction around a stress axis. Then it is known that a variation of a propagation velocity for example varies approximately linearly to a stress or a strain. By using the characteristic, with regard to shear waves propagating in two directions nonparallel with each other, approximate straight lines of velocities and stress indexes are calculated and the intersection is calculated. The intersection means the coincidence of acoustic characteristics, means a velocity in a stress-free state that cannot be reproduced in reality, and hence can be an elasticity evaluation index universal to an external force.

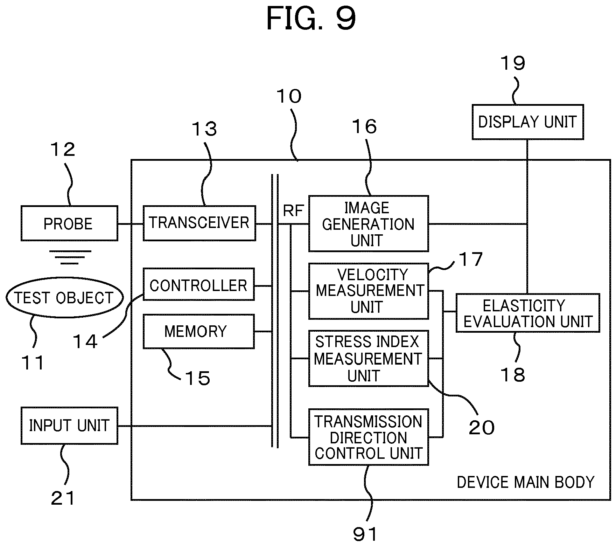

Example 2 is hereunder explained in detail in reference to drawings. Figure 9 shows a configuration example of an ultrasound diagnostic device according to Example 2. In the figure, except a transmission direction control unit 91, the processing contents of an image generation unit 16 to generate a B image used for setting a measurement region and a velocity measurement unit 17 to excite an acoustic radiation pressure in a test object and measure the propagation velocity of a shear wave are similar to Example 1 and the detailed explanations are omitted. Here, a velocity direction control unit 31 can also be operated by running a program in a CPU incorporated into a PC or the like as explained in Example 1.

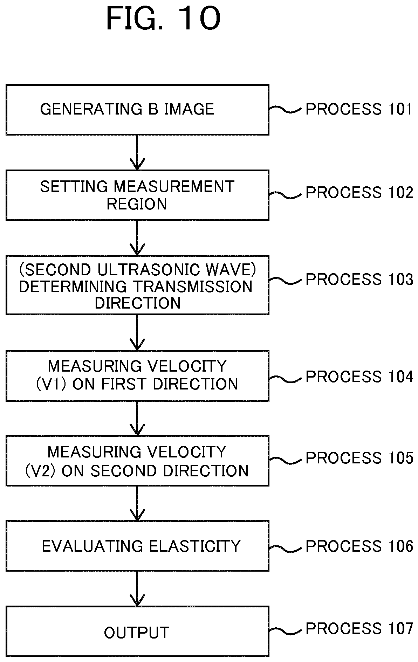

The contents of operation processing of a device according to the present example are explained in accordance with the flowchart shown in Figure 10. Generating a B image (Process 101) at the beginning and setting a measurement region (Process 102) based on the generated B image are identical to Example 1.

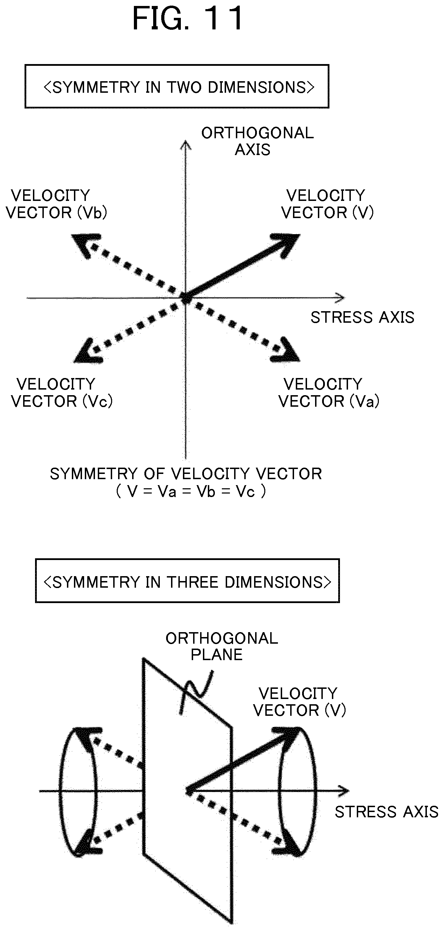

Successively, the transmission directions of a second ultrasonic wave to excite an acoustic radiation pressure are determined (Process 103). If an angle formed by a first direction and a second direction is defined as θ, firstly the condition 0 degree < θ ≤ 90 degrees has to be satisfied from an acoustic characteristic relative to an internal stress. This is a condition stipulated from the line symmetry (V = Va = Vb = Vc) of velocity vectors relative to a stress axis and an orthogonal axis in a two-dimensional space as shown at the upper stage in Figure 11, and from rotation symmetry relative to a stress axis and plane symmetry relative to an orthogonal plane in a three-dimensional space as shown at the lower stage. If θ is 0 degree for example, the velocity variations of the shear waves in the first and second directions caused by an internal stress are identical. If the first direction is set at 0 degree and θ in the second direction is set at 100 degrees, the acoustic characteristic of the shear wave propagating in the second direction is identical to that when θ is 80 degrees. Further, in order to maximize the difference of the acoustic characteristics of the shear waves propagating in two directions, it is effective to set θ at an angle as close to 90 degrees as possible.

Successively, a condition to be considered is an angle (steering range) transmittable by a probe 12. A steering range is stipulated basically as a range where the influence of a side lobe is not generated. That is, when a steering range is 90 degrees or more, the first direction and the second direction are determined by setting θ at 90 degrees. In contrast, when a steering range is less than 90 degrees, the first direction and the second direction are determined at both the ends of the steering range from the viewpoint of setting θ at a largest value possible.

Successively, at Process 104, a shear wave is generated by the transmission of the second ultrasonic wave in the first direction and subsequently a shear wave velocity V1 is measured by the transmission and reception of a third ultrasonic wave. Successively, at Process 105, a shear wave is generated by the transmission of the second ultrasonic wave in the second direction and subsequently a shear wave velocity V2 is measured by the transmission and reception of the third ultrasonic wave.

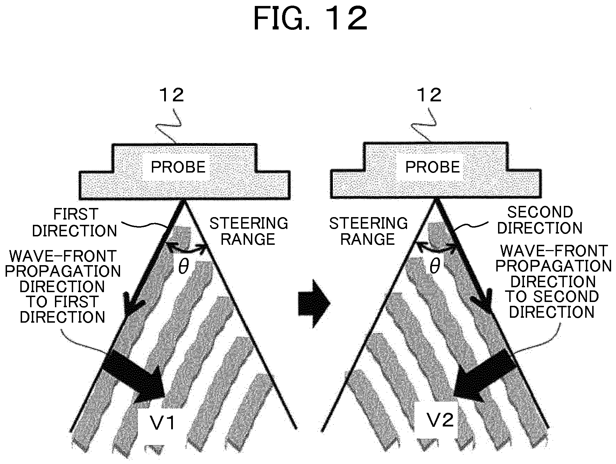

Figure 12 is an example of the first and second directions and the wave fronts of shear waves of V1 and V2 by the processing in Process 104 and Process 105. On this occasion, V1 and V2 are measured more than once alternately.

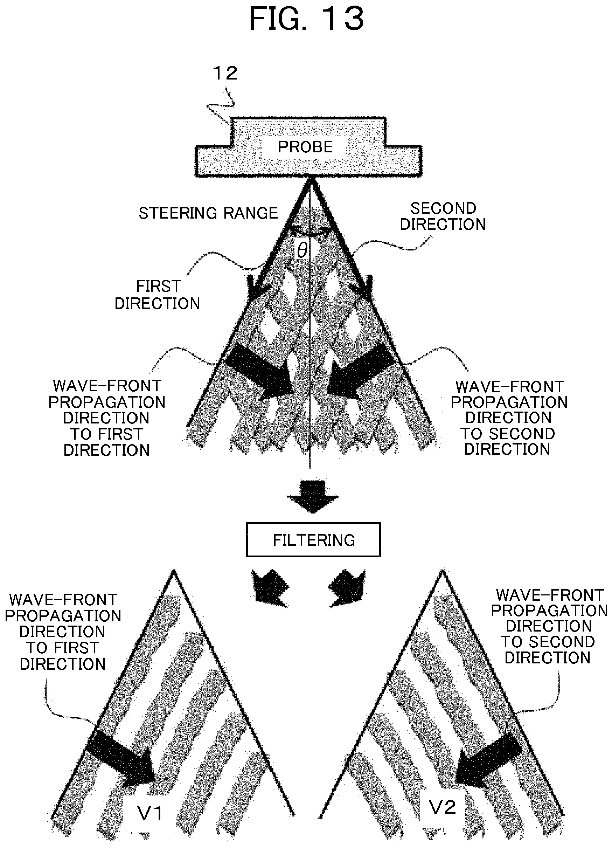

As shown in Fig. 13, as a method of measuring shear waves propagating in two directions, a method of transmitting first ultrasonic waves simultaneously in a first direction and a second direction is also conceivable. On this occasion, although shear waves propagating in the first and second directions are in a coexisting state, they can be separated by filtering treatment to extract a wave front propagating in a specific direction. By this method, a significant reduction of measurement time can be expected.

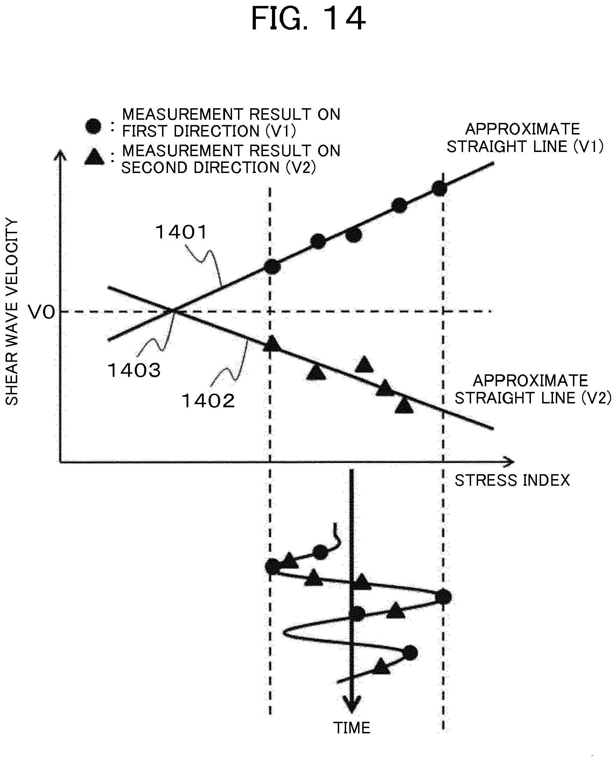

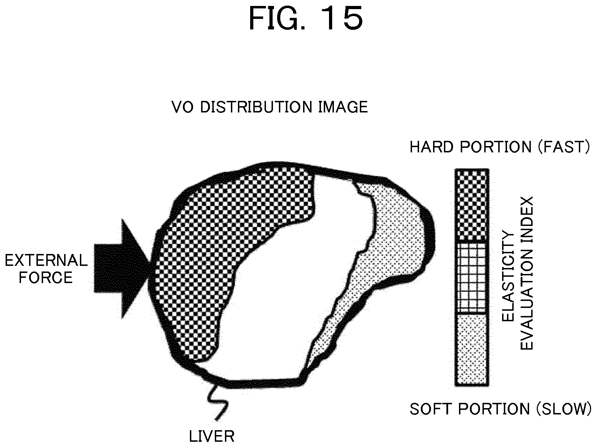

Elasticity is evaluated on the basis of the shear wave velocities V1 and V2 measured by the above method (Process 106). The result obtained by plotting the results of velocity measurement in contrast to stress indexes calculated by the method described in Example 1 is shown in Figure 14. Linear approximate straight lines 1401 and 1402 are fitted to the velocities V1 corresponding to the transmission in the first direction and the velocities V2 corresponding to the transmission in the second direction and the intersection 1403 is calculated. The stress index at the intersection 1403 represents a stress-free state (for example, a state where a stress or a strain is zero) and the propagation velocity V0 can be expressed as a stress-free velocity and is a numerical value of a shear wave velocity evading the influence of an internal stress. The propagation velocity V0 is outputted at Process 107 and displayed on a display unit 19. Then by setting measurement regions over a whole liver and measuring velocities in sequence, the distribution image of the velocity V0 shown in Figure 15 is configured. This is different from a conventional velocity distribution image shown in Figure 8A and is a velocity distribution image as an elasticity evaluation index evading the influence of an internal stress.

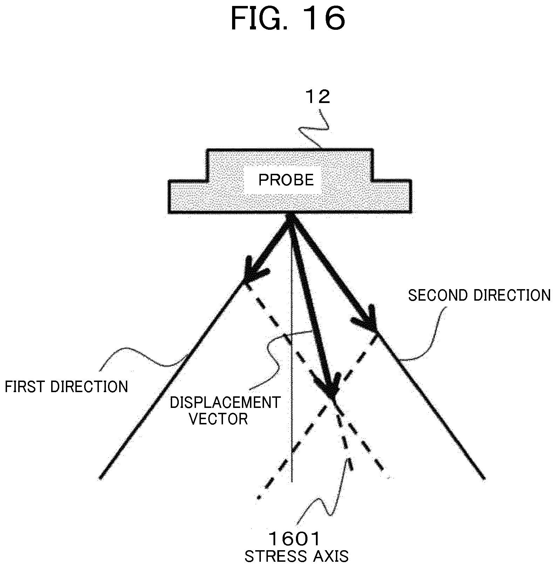

A stress index can be calculated by the method described in Example 1 but a method of using transmission toward two directions according to the present example is additionally conceivable. A first direction, a second direction, and a stress axis 1601 on which a maximum displacement is obtained are shown in Figure 16. By transmitting and receiving ultrasonic waves in the first direction and the second direction, component vectors of displacements in the respective directions accompanying an external force are measured. By summing up the measured component vectors, a displacement vector along the stress axis 1601 is measured.

In the present example, the positional relationship between the two directions where shear waves are generated and the stress axis is not taken into consideration. As shown in Figure 11, velocity vectors have point symmetry relative to a stress axis. That is, when a first direction and a second direction are set at equal angles in the manner of interposing a stress axis, no difference in acoustic characteristic appears between a velocity V1 and a velocity V2 and velocity calculation in a stress-free state is not realized. When such a situation appears in the execution of the present example, an elasticity evaluation index can be derived by measuring again while the angle is changed.

In this way, according to Example 2 described in detail, a shear wave velocity in a stress-free state evading the influence of an internal stress can be estimated and, by displaying it numerically or visually as an elasticity evaluation index, the provision of an ultrasound diagnostic device having an elasticity evaluation function of high degrees of accuracy and reproducibility can be realized.

### <Example 3>

Example 3 is an example of an ultrasound diagnostic device having an elasticity evaluation function of high degrees of accuracy and sensitivity by optimizing the transmission directions of a second ultrasonic wave to generate a shear wave in a method of deriving an elasticity evaluation index by two-direction transmission shown in Example 2. That is, a device according to the present example is configured so that the device further comprises a stress axis identification unit 171 the stress axis identification unit 171 identifies a stress axis allowing a variation of a stress index caused by an external force to be maximum by using a first or third ultrasonic wave, and a stress index measurement unit 20 measures the stress index on the stress axis. Further, the device is configured so that the transmission directions of a second ultrasonic wave determined by a transmission direction control unit 91 are optimized by using the stress axis and a transmittable range of a probe 12.

As shown in Figure 11, in a test object subjected to an internal stress, the velocity vectors of shear waves have point symmetry relative to a stress axis. That is, by identifying a stress axis beforehand, a direction optimum for calculating a stress-free velocity V0 can be determined.

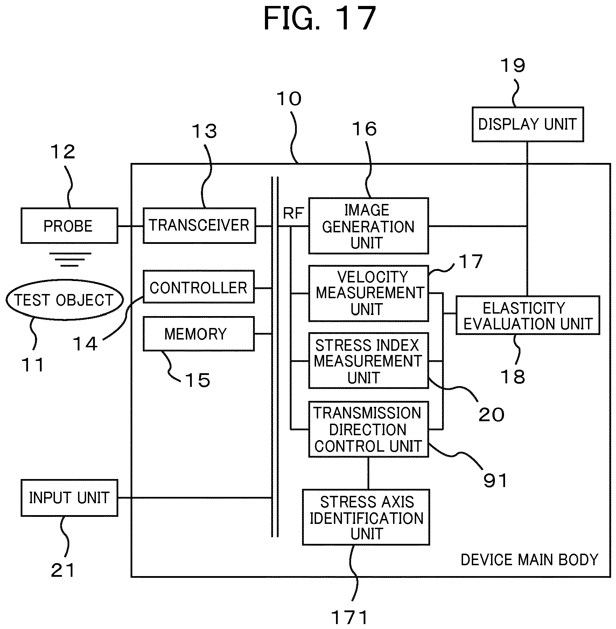

A configuration example of an ultrasound diagnostic device according to the present example is shown in Figure 17. The device is configured by adding a stress axis identification unit 171 to the device configuration according to Example 2 shown in Figure 9. The processing contents of generating a B image, generating a shear wave, and measuring a propagation velocity by the transmission of first, second, and third ultrasonic waves are identical to Example 1 or 2 explained earlier. The explanations of the present example therefore give importance to the processing from identifying a stress axis by a stress axis identification unit 171 to determining transmission directions by a transmission direction control unit 91 using the stress axis. Here, the stress axis identification unit 171 can also be operated by running a program in a CPU incorporated into a PC or the like.

Firstly, with regard to identifying a stress axis by the stress axis identification unit 171, as a method of configuring a displacement vector map of a test object based on a B image, various methods, starting with a speckle tracking method, have already been proposed and a stress axis can be identified by any of the methods. When a tissue under pulsation is an object for example, a displacement vector map in a setup measurement region is measured over a pulsation cycle. From the measurement result, a direction of generating a maximum displacement vector or an average direction of displacement vectors is calculated and can be identified as a stress axis for example.

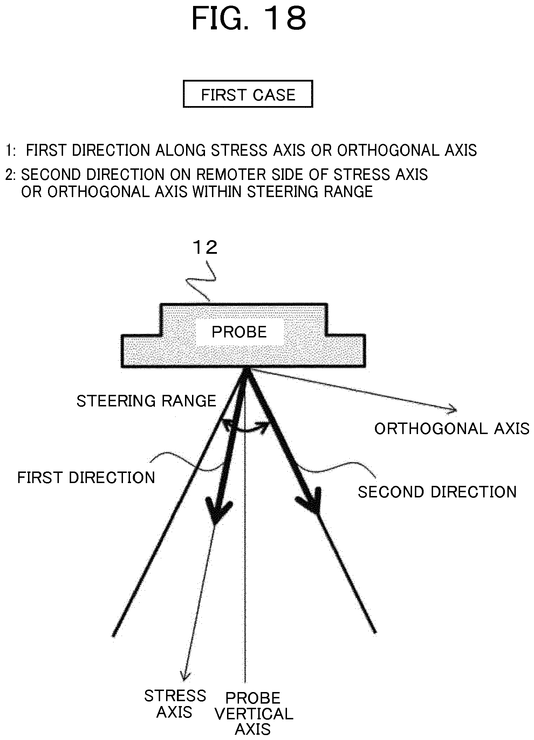

As another simple method, a method of transmitting and receiving ultrasonic waves in first and second two directions nonparallel with each other as shown in Figure 16 is also practical. A synthetic vector of component vectors (projected components of a displacement vector) in respective directions is defined as a displacement vector and a stress axis 1601 is identified by a method similar to the above methods. The method can combine with the velocity measurement of a shear wave as explained in Figure 6B of Example 1 and is efficient from the viewpoint of continuously monitoring a stress axis. Successively, as shown in Figures 18 to 20, in consideration of an identified stress axis, an orthogonal axis perpendicular to the stress axis, and the steering range of a probe 12, the transmission directions of a second ultrasonic wave optimum to the calculation of a stress-free velocity are determined. That is, the transmission directions of a second ultrasonic wave are optimized by using the stress axis and the transmittable range of a probe.

The first case shown in Figure 18 is a case of including either of a stress axis or an orthogonal axis in the steering range of a probe 12. Firstly, a first direction is set along a stress axis or an orthogonal axis included in a steering range. Successively, with the first direction used as a starting point, a second direction is set at the remoter end in both the ends of the steering range.

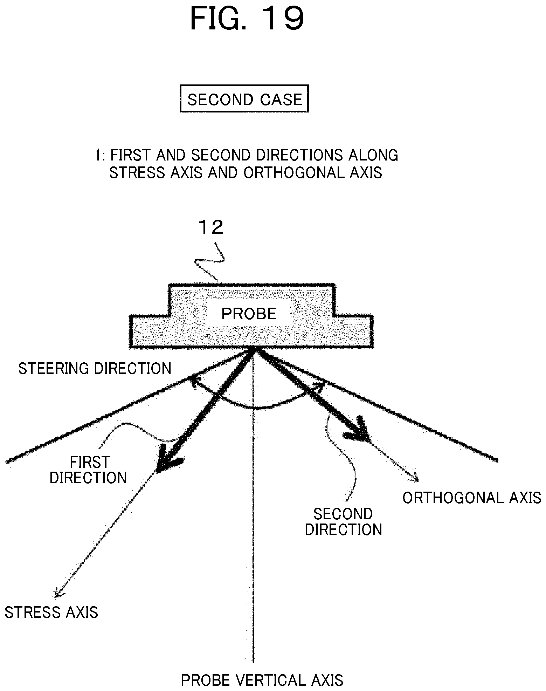

The second case shown in Figure 19 is a case of including both of a stress axis and an orthogonal axis in the steering range of a probe 12. On this occasion, a first direction and a second direction are set along the stress axis and the orthogonal axis.

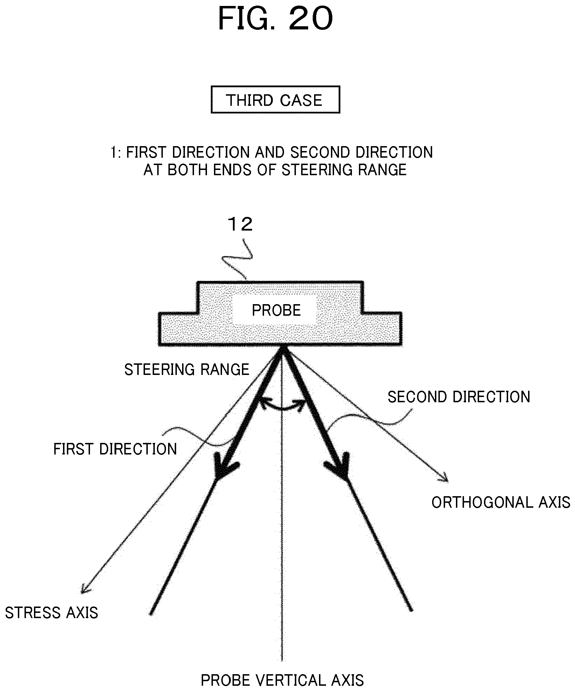

The third case shown in Figure 20 is a case of excluding both of a stress axis and an orthogonal axis from the steering range of a probe 12. On this occasion, a first direction and a second direction are set at both the ends of the steering range.

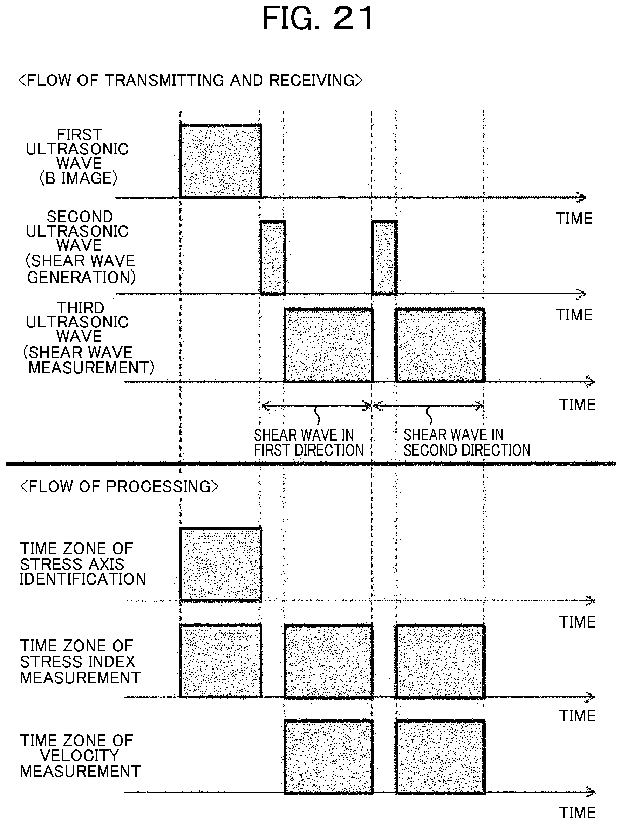

Finally, an example of a timing chart showing the correlation among the flow of the transmission and reception of a first, second, and third ultrasonic waves, the identification of a stress axis, the measurement of a stress index such as a displacement for example, and the velocity measurement of a shear wave is shown in Figure 21. As explained also in Figure 6B, a stress index can always be measured except the transmission time zone of a second ultrasonic wave, namely the time zone of generating a shear wave caused by the excitation of an acoustic radiation pressure, as shown in Figure 21.

By determining the optimum transmission directions of a second ultrasonic wave on the basis of the present example, the difference of the variations of the velocity V1 corresponding to the first direction and the velocity V2 corresponding to the second direction from the variation of a stress index is maximized and the calculation accuracy of a stress-free velocity V0 improves. Further, the identification of a stress axis is useful also for a high sensitivity measurement of a stress index and the maximum displacement can be an object by bringing the region of displacement measurement as close to a stress axis as possible.

In this way, according to Example 3 described in detail, a shear wave velocity in a stress-free state evading the influence of an internal stress can be estimated with high degrees of accuracy and sensitivity and, by displaying it numerically or visually as an elasticity evaluation index, the provision of an ultrasound diagnostic device and an elasticity evaluation method having an elasticity evaluation function of high degrees of accuracy and reproducibility can be realized.

### <Example 4>

Example 4 is an example of an ultrasound diagnostic device having an elasticity evaluation function allowing viscoelasticity to be evaluated by frequency analysis in a method of deriving an elasticity evaluation index shown in the above examples. A device according to the present example is configured so that the device further comprises a frequency analysis unit 221, and the frequency analysis unit 221 subjects the waveform of a measured shear wave to frequency analysis and outputs an index having frequency dependency. Further, the device is configured so that an elasticity evaluation unit 18 obtains a stress-free phase velocity by deriving a relationship between a propagation velocity and a frequency not susceptible to a stress index from a propagation velocity, a stress index, and an index having frequency dependency on the basis of the outputs of a velocity measurement unit 17, a stress index measurement unit 20, and a frequency analysis unit 221, and derives and outputs an elasticity evaluation index involved in the viscoelasticity of a test object on the basis of the frequency dependency of the stress-free phase velocity.

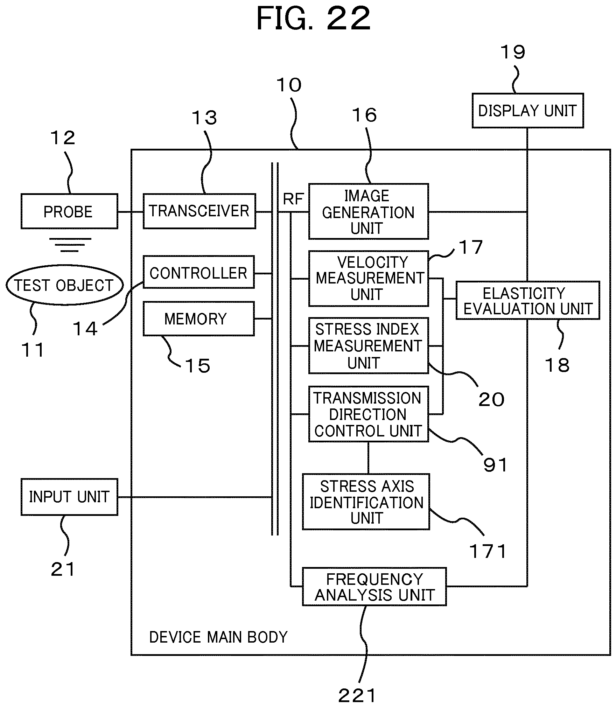

A configuration example of an ultrasound diagnostic device according to the present example is shown in Figure 22. As it is obvious in the figure, the device is configured by adding a frequency analysis unit 221 to the device configuration according to Example 3 shown in Figure 17. The processing contents of generating a B image, generating a shear wave, measuring a propagation velocity, and identifying a strain axis by the transmission of first, second, and third ultrasonic waves are identical to Example 1, 2, or 3 explained earlier. The explanations of the present example therefore give importance to the processing content of an elasticity evaluation method of analyzing the relationship between a measured propagation velocity and a measured frequency and deriving an elasticity evaluation index involved in the viscoelasticity of a test object on the basis of the analysis result. Here, the frequency analysis unit 221 can also be operated by processing a program in a CPU in a PC or the like in the examples stated above.

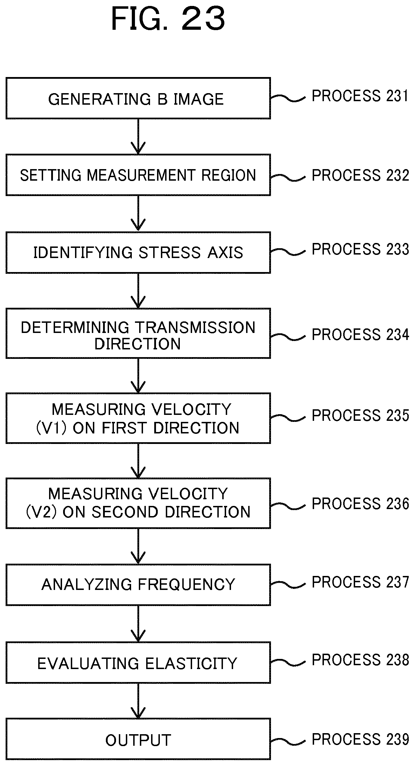

An example of an operation flowchart of a device according to the present example is shown in Figure 23. Firstly, a B image is generated by a first ultrasonic wave (Process 231), a measurement region where a shear wave is generated is set on the basis of the B image (Process 232), a stress axis is identified on the basis of the B image (namely a first ultrasonic wave transmission and reception signal) in the measurement region (Process 233), a first direction and a second direction in which a second ultrasonic wave is transmitted are determined on the basis of the stress axis and the steering range of a probe 12 (Process 234), the velocity (V1) of a shear wave related to the first direction is measured more than once (Process 235), and successively the velocity (V2) of a shear wave related to the second direction is measured more than once (Process 236).

Then in the present example, the frequency analysis unit 221 applies frequency analysis to the measurement result (V1 and V2) (Process 237), evaluates elasticity from the result of the frequency analysis (Process 238), and outputs the elasticity evaluation result to a display unit 19 (Process 239).

The frequency analysis by the frequency analysis unit 221 is carried out on the basis of a transmitted and received signal of a third ultrasonic wave. The number of measurement points located at short intervals in the propagation direction of a shear wave is desirably large in order to enhance the accuracy of the frequency analysis. By applying frequency analysis to a shear wave propagating into a test object, a numerical value, such as a phase velocity or a frequency-dependent attenuation, obtained from the frequency analysis is calculated as an index of a propagation velocity having the frequency dependency. In general, a phase velocity is information effective for evaluating the viscosity of a test object and velocity variation is more conspicuous in a high-frequency zone as the viscosity of a medium increases. Likewise with regard to attenuation too, the attenuation is more conspicuous in a higher-frequency zone and hence a numerical value related to frequency-dependent attenuation is also effective information.

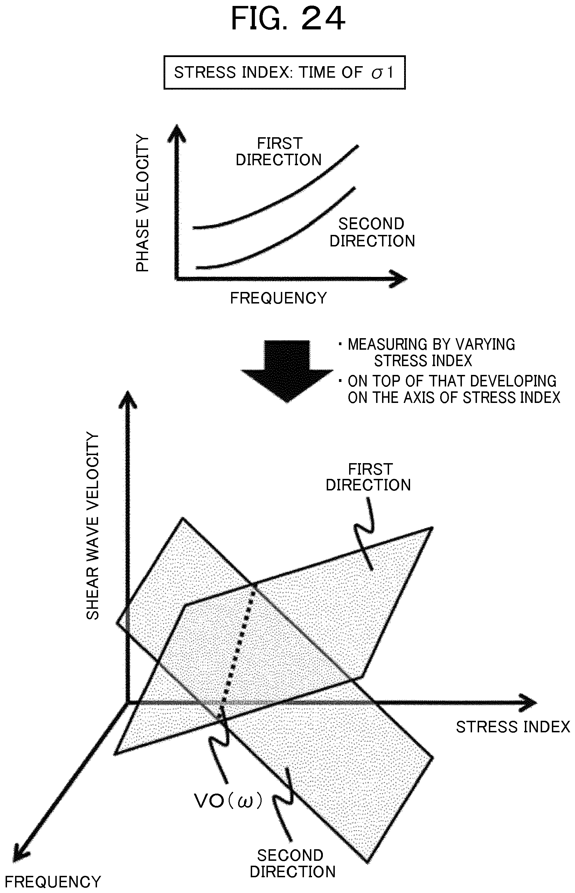

A graph of the relationship between a phase velocity and a frequency corresponding to each of the velocities V1 and V2 in the first and second directions in a certain stress state (when a stress index is σ1) is exemplified at the upper stage in Figure 24. The relationship graph is the information obtained as a result of frequency analysis by the frequency analysis unit 221. By varying a stress index while the information is kept in mind, measuring V1 and V2 in the states of a plurality of stress indexes, and applying frequency analysis to the results, a three-dimensional graph in which a frequency axis is added in addition to the axes of a shear wave velocity and a stress index is configured as exemplified at the lower stage in Figure 24. As it is obvious from the graph exemplified at the lower stage in the figure, each of the velocity (V1) in the first direction and the velocity (V2) in the second direction is drawn as a plane and the line of intersection is calculated as a phase velocity V0(ω) (when f is defined as a frequency, ω = 2nf) in a stress-free state V0.

The above explanations have been made on the basis of a phase velocity V0(ω) in a stress-free state V0 and, as information obtained by an elasticity evaluation unit 18 according to the present example from a propagation velocity, a stress index, and an index having frequency dependency (a numerical value such as a phase velocity or a frequency-dependent attenuation obtained from waveform analysis) that are the outputs from a velocity measurement unit 17, a stress index measurement unit 20, a frequency analysis unit 221, and the like, a group velocity, a phase velocity, a complex modulus (storage elastic modulus and loss elastic modulus), and a frequency-dependent attenuation in a stress-free state are mentioned. All of those are elasticity evaluation indexes related to the elasticity of a test object and to display them as a numerical value, a graph, or a two-dimensional map of each of them on a display unit 19 is effective as diagnostic information to support the qualitative diagnosis of the test object.

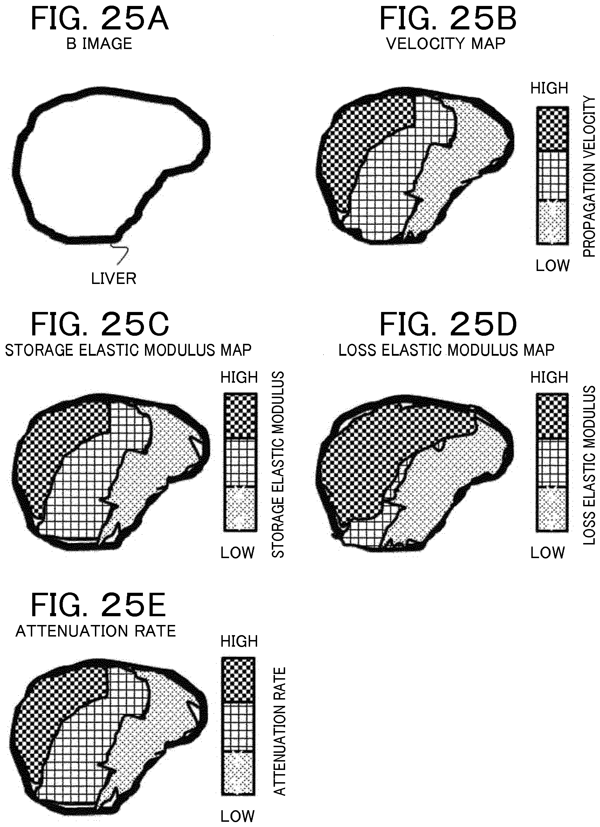

Two-dimensional maps of the elasticity evaluation indexes displayed on a display unit in a device according to the present example are shown schematically in Figure 25. (a) in Figure 25 represents a B image of a liver, (b) in Figure 25 represents a velocity map (numerical values representing the propagation velocity of a shear wave such as a group velocity, a phase velocity, or a stress-free velocity), (c) in Figure 25 represents a storage elastic modulus map (the real part of a complex modulus), (d) in Figure 25 represents a loss elastic modulus map (the imaginary part of a complex modulus), and (e) in Figure 25 represents an attenuation rate (such as a frequency-dependent attenuation). In this way, various results of elasticity evaluation derived by an elasticity evaluation unit 18 are outputted to and displayed on the display unit 19 in the form of a numerical value, a graph, or an image. Here, it goes without saying that two-dimensional maps of the various results of elasticity evaluation on the display unit 19 can be displayed not only in the present example but also in another example. That is, the display unit 19 is used for displaying an elasticity evaluation index derived by an elasticity evaluation unit as a numerical value, a graph, or an image in each of the examples.

According to Example 4, the provision of an ultrasound diagnostic device having an elasticity evaluation function of high degrees of accuracy and reproducibility can be realized by calculating the phase velocity of a shear wave in a stress-free state where the influence of an internal stress is evaded and displaying an elasticity evaluation result related to viscoelasticity and obtained through frequency analysis in the form of each or a combination of a numerical value, a graph, and a two-dimensional map.

As a result of the present invention explained above in detail, by deriving an elasticity evaluation index based on velocity variation caused by difference in internal stress of a test object, a measurement error caused by a region or a time phase is mitigated, elasticity evaluation of high degrees of accuracy and reproducibility is realized, and an accurate qualitative diagnosis by an ultrasonic wave is expected.

In the case of a disease advancing chronically in particular, medium- and long-term observation on tissue elasticity is required on the occasion of deciding a therapeutic intervention or judging a therapeutic effect and the accuracy and reproducibility in the present invention result in a clinically useful effect.

Further, in the case of assuming a tumor, an internal pressure increases frequently in the interior and periphery of a tumor by the influence of tumor hypertrophy in the state of before treatment but, by the present invention, a qualitative evaluation of the tumor can be expected by evaluating difference in internal pressure between the interior or periphery of the tumor and a normal region. Further, an accurate judgment of a therapeutic effect can be expected by quantitatively evaluating the variation of a tumor inner pressure likewise before and after treatment.

The various embodiments according to the present invention have heretofore been explained but the present invention is not limited to the above embodiments and includes various modified examples. For example, the embodiments stated above are explained in detail for a better comprehension of the present invention and the present invention is not necessarily limited to a case having all the explained configurations. Further, it is possible to replace a part of the configuration of an embodiment with the configuration of another embodiment, and also add the configuration of an embodiment to the configuration of another embodiment. For example, a signal processing unit having both the configuration in Figure 1 and the configuration in Figure 2 can be used. Furthermore, it is also possible to add, delete, or replace a part of the configuration of each of the embodiments to, from, or with another configuration.

Moreover, although the configurations, functions, processing units, and others stated above are explained on the basis of an example of using a CPU in a PC or the like running a program for operating a part or the whole of each of them, it goes without saying for example that the part or the whole may be operated with hardware for exclusive use by designing an IC for example specialized in the functions or the like.

In the various examples described above in detail, not only the invention described in the scope of the claims but also various kinds of invention are disclosed. Some of them are listed below.

### <List 1>

An elasticity evaluation method comprising the processes of
transmitting and receiving first, second, and third ultrasonic waves to and from a test object through a probe to transmit and receive an ultrasonic wave,
generating an image of the test object by a received signal based on the first ultrasonic wave,
generating a shear wave by transmitting the second ultrasonic wave into a measurement region set on the basis of the image,
measuring the propagation velocity of the shear wave by the third ultrasonic wave more than once at an identical site, and
deriving an elasticity evaluation index of the test object by using the variation of the obtained propagation velocity measurement results.

### <List 2>

An elasticity evaluation method described in List 1, comprising the process of measuring the variation of the test object accompanying an external force as a stress index by using a received signal based on the transmission of the first ultrasonic wave or the third ultrasonic wave.

### <List 3>

An elasticity evaluation method described in List 2, comprising the process of measuring a stress index by the third ultrasonic wave in a region where the third ultrasonic wave propagates except a region where the shear wave propagates.

### <List 4>

An elasticity evaluation method described in List 3, comprising the processes of evaluating the relationship between a plurality of measured propagation velocities and the stress indexes corresponding to the respective propagation velocities with an approximate straight line, and defining an inclination angle or a velocity variation of the approximate straight line as the elasticity evaluation index.

### <List 5>

An ultrasound diagnostic device comprising
an elasticity evaluation unit to evaluate the elasticity of a test object on the basis of the propagation velocity measured by a velocity measurement unit to generate a shear wave in the measurement region of the test object and measure the propagation velocity of the shear wave by an ultrasonic wave and
a stress index measurement unit to measure the variation of the test object accompanying an external force as a stress index by using a received signal based on the transmission of the ultrasonic wave, wherein
the velocity measurement unit measures velocities more than once at an identical site, and
the elasticity evaluation unit derives an elasticity evaluation index of the test object by using the variation of the velocity measurement results and the stress index.

### Reference Signs List

10: device main body
11: test object
12: probe
13: transceiver
14: controller
15: memory
16: image generation unit
17: velocity measurement unit
18: elasticity evaluation unit
19: display unit
20: stress index measurement unit
21: input unit
31: measurement region
32: stress axis
41: ECG waveform
51: pulsation cycle and waveform of velocity variation
61: wave-front measurement region
62: displacement measurement region
63: radiation pressure distribution
64: wave-front propagation range
65, 66, 67: time zone
71, 1401, 1402: approximate straight line
91: transmission direction control unit
171: stress axis identification unit
221: frequency analysis unit
1601: stress axis

## Claims

1. An ultrasound diagnostic device comprising
a transceiver to transmit and receive first, second, and third ultrasonic waves to and from a test object through a probe to transmit and receive an ultrasonic wave,
an image generation unit to generate an image of the test object by a received signal based on the first ultrasonic wave,
a velocity measurement unit to transmit the second ultrasonic wave to a measurement region set on the basis of the image, generate a shear wave, and measure the propagation velocity of the shear wave by the third ultrasonic wave, and
an elasticity evaluation unit to evaluate the elasticity of the test object on the basis of the propagation velocity, wherein the velocity measurement unit measures the velocity more than once at an identical site, and
the elasticity evaluation unit derives an elasticity evaluation index of the test object by using the variation of the velocity measurement results.

2. An ultrasound diagnostic device according to Claim 1, wherein the ultrasound diagnostic device further comprises a stress index measurement unit, and
the stress index measurement unit measures the variation of the test object accompanying an external force as a stress index by using a received signal based on the transmission of the first ultrasonic wave or the third ultrasonic wave.

3. An ultrasound diagnostic device according to Claim 2, wherein the stress index measurement unit derives the stress index on the basis of an electrocardiographic waveform of the test object.

4. An ultrasound diagnostic device according to Claim 2, wherein the stress index measurement unit measures the stress index using the third ultrasonic wave on the basis of a signal received from a region obtained by excluding a region where the shear wave propagates from a region where the third ultrasonic wave propagates.

5. An ultrasound diagnostic device according to Claim 2, wherein the elasticity evaluation unit evaluates the relationship between a plurality of the propagation velocities measured by the velocity measurement unit and the stress indexes corresponding to respective the propagation velocities with an approximate straight line, and defines an inclination angle or a velocity variation of the approximate straight line as the elasticity evaluation index.

6. An ultrasound diagnostic device according to Claim 2, wherein the ultrasound diagnostic device further comprises a transmission direction control unit to control a transmission direction of the ultrasonic wave,
the transmission direction control unit determines a plurality of directions not parallel with each other and transmits the second ultrasonic wave to the directions, and
the velocity measurement unit measures the propagation velocities of the shear waves corresponding to transmission in the directions.

7. An ultrasound diagnostic device according to Claim 6, wherein the elasticity evaluation unit evaluates relationship between a plurality of the propagation velocities measured by the velocity measurement unit and the stress indexes corresponding to respective the propagation velocities with approximate straight lines, and outputs a velocity numerical value at the intersection of the approximate straight lines involved in respective propagation directions as the elasticity evaluation index.

8. An ultrasound diagnostic device according to Claim 6, wherein the ultrasound diagnostic device further comprises a stress axis identification unit;
the stress axis identification unit identifies a stress axis allowing a variation of the stress index caused by an external force to be maximum by using the first or third ultrasonic wave, and
the stress index measurement unit measures the stress index on the stress axis.

9. An ultrasound diagnostic device according to Claim 8, wherein the transmission directions of the second ultrasonic wave determined by the transmission direction control unit are optimized by using the stress axis and a transmittable range of the probe.

10. An ultrasound diagnostic device according to Claim 2, wherein the ultrasound diagnostic device further comprises a frequency analysis unit, and
the frequency analysis unit subjects the waveform of measured the shear wave to frequency analysis and outputs an index having frequency dependency.

11. An ultrasound diagnostic device according to Claim 10, wherein
the elasticity evaluation unit obtains a stress-free phase velocity by deriving a relationship between the propagation velocity and a frequency not susceptible to a stress index on the basis of the outputs of the velocity measurement unit, the stress index measurement unit, and the frequency analysis unit, and derives an elasticity evaluation index involved in the viscoelasticity of the test object on the basis of the frequency dependency of the stress-free phase velocity.

12. An ultrasound diagnostic device according to Claim 1, wherein the ultrasound diagnostic device further comprises a display unit; and
the display unit displays the elasticity evaluation index derived by the elasticity evaluation unit as a numerical value,
a graph, or an image.

13. An ultrasound diagnostic device according to Claim 1, wherein the elasticity evaluation index is numerical information that is involved in elasticity of a material, and obtained from a displacement, a strain, a particle velocity, a group velocity, a phase velocity, an attenuation rate, a Young's modulus, a rigidity modulus, a complex modulus (storage elastic modulus, loss elastic modulus), a volume elasticity modulus, a shear wave velocity, a longitudinal wave velocity, a viscosity rate, or a Poisson's ratio of the test object.

14. An elasticity evaluation method using a probe, comprising the processes of:
generating a shear wave in a measurement region of a test object;
measuring the propagation velocity of the shear wave in the measurement region more than once at an identical site by transmitting and receiving an ultrasonic wave to and from the probe, and
deriving an elasticity evaluation index of the test object by using the variation of a plurality of obtained velocity measurement results.

15. An elasticity evaluation method according to Claim 14, comprising
the process of measuring variation of the test object accompanying an external force as a stress index by using a received signal based on the transmission of the first ultrasonic wave or the third ultrasonic wave.
